# FASCICULE DE BREVET EUROPEEN

(11) **EP 2 676 658 B1**
(45) Date de publication et mention de la délivrance du brevet: **16.09.2015**
(21) Numéro de dépôt: 13184851.7
(22) Date de dépôt: 04.11.2009
(51) Int. Cl.: A61K 9/00, A61K 31/00, A61L 27/00

(54) **Composition injectable à base d'acide hyaluronique, de polyol(s) et de lidocaïne, stérilisée à la chaleur**
Hitzesterilisierte Injizierbare Zusammensetzung auf der Basis von Hyaluronsäure, Polyol(en) und Lidocain
Heat-sterilised injectable composition of hyaluronic acid, polyol(s) and lidocaine

(30) Priorité: 07.11.2008 FR 0857575
(43) Date de publication de la demande: 25.12.2013
(62) Demande divisionnaire de: 09768146.4
(73) Titulaire: Anteis SA, 1228 Plan-Les-Ouates (CH)
(72) Inventeur: Gavard Molliard, Samuel, 74250 BOGEVE (FR)
(74) Mandataire: Ricker, Mathias

(56) Documents cités:
- WO-A-98/41171
- WO-A-2007/077399
- WO-A-2008/068297
- WAHL, G.: "European evaluation of a new hyaluronic acid filler incorporating lidocaine", JOURNAL OF COSMETIC DERMATOLOGY, vol. 7, 6 novembre 2008 (2008-11-06), pages 298-303, XP002516376,

## Description

L'invention concerne une composition aqueuse injectable sous forme de gel à base d'acide hyaluronique ou l'un de ses sels, d'un ou plusieurs polyol(s) et de lidocaïne, composition stérilisée à la chaleur présentant des propriétés rhéologiques améliorées et une longue rémanence in vivo, pour une utilisation dans des buts esthétiques ou dans des buts thérapeutiques.

Les gels injectables à base d'acide hyaluronique sont utilisés depuis de nombreuses années dans des buts esthétiques pour le comblement ou le remplacement de tissus biologiques (comblement des rides, remodelage du visage, augmentation du volume des lèvres, ...) ainsi que dans le traitement de réhydratation de la peau par mésothérapie.

Les gels injectables à base d'acide hyaluronique sont également utilisés dans de nombreuses applications thérapeutiques. Par exemple,
en rhumatologie, en tant qu'agent de remplacement, de supplément temporaire du liquide synovial,
en urologie/gynécologie, en tant qu'agent permettant d'augmenter le volume du sphincter ou de l'urètre,
en ophtalmologie, en tant qu'adjuvant à la chirurgie de la cataracte ou pour le traitement des glaucomes,
en pharmaceutique, en tant que gel à libération de substances actives,
en chirurgie, pour la reconstruction osseuse, l'augmentation du volume des cordes vocales ou la fabrication de tissus chirurgicaux.

De nombreux efforts ont été faits pour améliorer la stabilité physico-chimique des gels à base d'acide hyaluronique afin d'augmenter leur rémanence in vivo (c'est-à-dire le temps de résidence du gel au niveau du site d'injection) et ainsi, augmenter la durée d'efficacité des traitements.

D'après l'art antérieur, l'augmentation de la rémanence des gels à base d'acide hyaluronique et donc de leur résistance aux différents facteurs de dégradation in vivo est essentiellement mise en oeuvre par des techniques de réticulation et/ou greffage de l'acide hyaluronique. Par exemple,
- WO 2005/012364 décrit des gels à base de polysaccharides, dont l'acide hyaluronique, réticulés et greffés, ayant une meilleure rémanence que les produits non réticulés et/ou non greffés.
- WO 2004/092222 décrit des gels à base de polysaccharides, dont l'acide hyaluronique, renfermant au moins un polysaccharide de faible masse moléculaire et au moins un polysaccharide de forte masse moléculaire, gels ayant une rémanence supérieure à celle de produits ne possédant pas de mélange de masses moléculaires.
- WO 2005/085329 décrit un procédé de fabrication permettant l'obtention de gels à base d'acide hyaluronique réticulé polydensifié, gels possédant une longue rémanence in vivo.
- WO 0046252 décrit des gels à base d'acide hyaluronique à forte « biostabilité » possédant un haut degré de réticulation grâce à un procédé de double réticulation de l'acide hyaluronique.

Les polyols appartiennent à une famille de molécules de formule chimique CₓH_{y}O_{z} qui possède au moins deux groupements alcool. De par leur forte capacité à ajuster l'osmolarité, l'homme de l'art sait qu'il peut introduire des polyols dans une formulation aqueuse injectable afin d'obtenir une composition iso-osmolaire.

La lidocaïne (ou chlorhydrate de lidocaïne), est un anesthésique local couramment utilisé dans les domaines esthétique et médical. Cette molécule est notamment utilisée depuis de nombreuses années dans des produits à visée esthétique comme les produits de comblement des rides afin de limiter la douleur au cours et après injection (cas du produit Zyderm® contenant du collagène et 0,3% de lidocaïne).

Dans l'art antérieur, sont décrits des gels à base d'acide hyaluronique pouvant contenir un polyol et/ou de la lidocaïne. Par exemple,
- WO 2007/077399 décrit des gels à base d'acide hyaluronique et d'un alcool biocompatible visqueux dont la stérilisation augmente la viscosité.
- WO 2004/032943 décrit des gels à base d'acide hyaluronique contenant des anesthésiques locaux dont la lidocaïne.
- WO 98/41171 décrit une composition injectable sous forme de gel à base d'acide hyaluronique de mannitol et de lidocaïne.
- WO 2008/068297 décrit l'emploi d'un implant injectable par voie sous-cutanée ou intradermique sous forme d'hydrogel d'acide hyaluronique. Il divulgue également l'utilisation du mannitol comme agent anti-oxydant.
- European evaluation of a new hyaluronic acid filler incorporating lidocaine, G. WAHL, Journal of Cosmetic Dermatology, Vol. 7, 6, Novembre 2008, pages 298-303, décrit une formulation à usage dermatologique comprenant de l'acide hyaluronique et de la lidocaïne.

On a maintenant découvert que l'ajout d'un polyol et de lidocaïne dans un gel à base d'acide hyaluronique, réticulé, greffé ou non greffé, suivi d'une stérilisation de cette formulation à la chaleur, permet (par rapport à un gel sans polyol et sans lidocaïne) :
une amélioration très importante des propriétés rhéologiques du gel
une amélioration de la rémanence du gel en contrant les trois grands types de dégradation d'un gel à base d'acide hyaluronique in vivo (dégradation enzymatique par les hyaluronidases, dégradation radicalaire, dégradation thermique à 37°C)
une amélioration de la stabilité rhéologique du gel au cours du temps et donc une durée de péremption du produit pouvant être allongée.

Il a en effet été montré, que de façon tout à fait surprenante, l'ajout d'un ou plusieurs polyol(s) et de lidocaïne dans un gel à base d'acide hyaluronique :
- ne modifie pas les propriétés rhéologiques du gel avant stérilisation à la chaleur
- modifie considérablement les propriétés rhéologiques du gel après stérilisation à la chaleur (par rapport à un gel sans polyol et sans lidocaïne).

En d'autres termes, avant stérilisation à la chaleur, les propriétés viscoélastiques d'un gel à base d'acide hyaluronique avec polyol et lidocaïne sont identiques à celles d'un gel à base d'acide hyaluronique sans polyol et sans lidocaïne.

Après stérilisation à la chaleur, les propriétés viscoélastiques d'un gel à base d'acide hyaluronique avec polyol et lidocaïne sont différentes de celles d'un gel à base d'acide hyaluronique sans polyol et sans lidocaïne. Le gel avec polyol et lidocaïne possède une plus forte élasticité (G' plus élevé) et un caractère viscoélastique plus élastique (Tanδ plus faible) par rapport à un gel sans polyol et sans lidocaïne.

La stérilisation à la chaleur modifie profondément la structure du gel et donc les propriétés viscoélastiques de celui-ci (diminution des paramètres rhéologiques G' et G" / augmentation du paramètre Tanδ). On constate que la présence d'un polyol et de la lidocaïne dans un gel à base d'acide hyaluronique modifie considérablement l'évolution des paramètres rhéologiques lors de la stérilisation à la chaleur (limitation de l'évolution des paramètres rhéologiques : diminution du G' et du G" significativement moins forte / augmentation du Tanδ significativement moins forte).

En limitant la dégradation du gel à base d'acide hyaluronique lors de la stérilisation à la chaleur, la structure du gel à base d'acide hyaluronique avec polyol et lidocaïne est différente de celle obtenue avec un gel à base d'acide hyaluronique sans polyol et sans lidocaïne. Cette structure possède notamment un caractère élastique renforcée (meilleure capacité du gel à créer du volume).

Cette meilleure résistance à la dégradation thermique confère notamment au gel une meilleure stabilité du produit à température ambiante au cours du temps. Ainsi, un produit fabriqué selon cette invention possédera une durée de péremption qui pourra être allongée par rapport à un produit ne possédant pas de polyol et de lidocaïne.

Il a également été montré qu'un gel à base d'acide hyaluronique avec polyol et lidocaïne possède une meilleure résistance aux trois facteurs de dégradation d'un gel à base d'acide hyaluronique in vivo par rapport à un gel sans polyol et sans lidocaïne :
- meilleure résistance à la dégradation enzymatique
- meilleure résistance à la dégradation radicalaire
- meilleure résistance à la dégradation thermique

En particulier, l'amélioration de la résistance à la dégradation thermique et radicalaire est tout à fait remarquable.

Sans vouloir se lier à une explication théorique de l'effet du polyol et de la lidocaïne à l'encontre des dégradations d'un gel à base d'acide hyaluronique, on suppose que la lidocaïne accroit de façon considérable la capacité d'un polyol à protéger un gel à base d'acide hyaluronique.

D'autre part, le ou les polyol(s) incorporé(s) dans le gel à base d'acide hyaluronique peuvent migrer hors du gel.

Hors du gel, le ou les polyol(s) vont pouvoir diffuser dans les tissus et jouer un rôle important par exemple dans l'hydratation des tissus ou encore en intervenant dans des mécanismes cellulaires ou biochimiques.

Enfin, la présence de lidocaïne dans le gel présente un intérêt majeur pour l'amélioration du confort du patient au cours et après injection.

Par conséquent, la composition aqueuse injectable à base d'acide hyaluronique ou l'un de ses sels, d'un ou plusieurs polyol(s) et de lidocaïne selon l'invention présente les avantages suivants dans des buts esthétiques ou dans des buts thérapeutiques:
- meilleures propriétés rhéologiques du gel (notamment meilleure capacité à créer du volume) car significativement moins dégradé à la stérilisation
- plus grande rémanence du gel in vivo et donc un effet à plus long terme du traitement grâce à l'action du ou des polyol(s) et de la lidocaïne à l'encontre des trois principaux types de dégradation du gel in vivo
- meilleure stabilité rhéologique du gel au cours de sa durée de péremption
- action positive des polyols sur l'organisme (par exemple, hydratation des tissus)
- amélioration du confort du patient par l'effet anesthésiant de la lidocaïne au cours et après injection.

L'invention fournit donc une composition injectable stérilisée à la chaleur comprenant un gel à base d'acide hyaluronique ou l'un de ses sels, un ou plusieurs polyol(s) et de la lidocaïne.

Cette composition est utilisée :
- dans des buts esthétiques pour le comblement des tissus biologiques ou pour la réhydratation de la peau par mésothérapie
- dans des buts thérapeutiques comme par exemple :
   a) en rhumatologie, en tant qu'agent de remplacement, de supplément temporaire du liquide synovial,
   b) en urologie/gynécologie, en tant qu'agent permettant d'augmenter le volume du sphincter ou de l'urètre,
   c) en ophtalmologie, en tant qu'adjuvant à la chirurgie de la cataracte ou pour le traitement des glaucomes,
   d) en pharmaceutique, en tant que gel à libération de substances actives,
   e) en chirurgie, pour la reconstruction osseuse, l'augmentation du volume des cordes vocales ou la fabrication de tissus chirurgicaux.

Selon les modes de réalisation de l'invention, le gel à base d'acide hyaluronique ou l'un de ses sels est non réticulé ou réticulé.

Selon les modes de réalisation de l'invention, la concentration en acide hyaluronique ou l'un de ses sels est comprise entre 0,01 mg/ml et 100 mg/ml et la masse moléculaire de l'acide hyaluronique ou l'un de ses sels est comprise entre 1000 Da et 10×10⁶ Da.

Selon un mode de réalisation particulier de l'invention, la réticulation se fait par des molécules bi- ou poly-fonctionnelles choisies parmi les époxydes, les épihalohydrines et la divinylsulfone, sur de l'acide hyaluronique non réticulé ou déjà réticulé avec ou sans un ou plusieurs autres polysaccharides d'origine naturelle.

Les différents types de gels à base d'acide hyaluronique sont connus dans la technique, et les gels réticulés et greffés sont décrits par exemple dans WO 2005/012364.

Selon un mode particulier de l'invention, le gel peut également contenir d'autres polymères biocompatibles (comme des polysaccharides d'origine naturelle) et/ou d'autres substances actives ou non actives ayant un effet positif sur l'organisme ou sur le gel.

Selon la caractéristique de l'invention, ces gels contiennent un ou plusieurs polyol(s) choisi par exemple parmi le glycérol, le sorbitol, le propylène glycol, le xylitol, le mannitol, l'erythritol, le maltitol ou le lactitol.

Selon les modes de réalisation de l'invention, la concentration en polyol dans le gel est comprise entre 0,0001 et 500 mg/ml et plus particulièrement entre 0,0001 et 100 mg/ml.

Selon les modes de réalisation de l'invention, la concentration en lidocaïne dans le gel est comprise entre 0,0001 et 500 mg/ml et plus particulièrement entre 0,001 et 50 mg/ml

Selon les modes de réalisation de l'invention, la stérilisation est effectuée à la chaleur sèche ou humide, préférentiellement à la chaleur humide. L'homme de l'art saura sélectionner un cycle de stérilisation à la chaleur (température et durée du cycle de stérilisation) approprié à la stérilisation de son produit. Par exemple, les cycles de stérilisation à la chaleur humide suivants peuvent être utilisés : 131°C, 1 min / 130°C, 3 min / 125°C, 7 min / 121°C, 20 min / 121°C, 10 min / 100°C, 2h.

### EXEMPLES :

Les formulations préparées dans les exemples suivants sont des gels à base de hyaluronate de sodium (NaHA) réticulé avec ou sans polyol et lidocaïne dans une solution aqueuse tamponnée à pH=7.

La préparation des gels réticulés est effectuée selon les techniques bien connues par l'homme de l'art. Le hyaluronate de sodium utilisé pour fabriquer ces gels possède une masse moléculaire égale à 2,5×10⁶ Da. Le réticulant utilisé est le butanediol diglycidyle éther (BDDE) et la définition du taux de réticulation utilisée est : masse (BDDE) / masse (NaHA sec).

L'incorporation du polyol dans le gel est effectuée en ajoutant la quantité nécessaire de polyol (% exprimé en masse) dans le gel et en mélangeant à la spatule pendant 10 minutes (pour 50g de gel final).

L'incorporation de la lidocaïne dans le gel est effectuée en ajoutant la quantité nécessaire de lidocaïne (% exprimé en masse) dans le gel et en mélangeant à la spatule pendant 10 minutes (pour 50g de gel final).

Afin d'avoir des conditions de fabrication strictement identiques pour les gels avec et sans polyol et lidocaïne, le gel réticulé sans polyol et sans lidocaïne est également mélangé à la spatule pendant 10 minutes (pour 50g de gel final).

Les gels préparés sont remplis en seringue verre puis stérilisés à la chaleur (121°C, 10 min).

Le rhéomètre utilisé pour effectuer les caractérisations rhéologiques est un AR2000 (TA instruments) avec une géométrie plate de 40 mm, un entrefer de 1000 µm et une température d'analyse de 37°C.

### Exemple 1 : Mise en évidence par la rhéologie de la différence de structure après stérilisation à la chaleur entre des gels à base d'acide hyaluronique avec et sans polyol/lidocaïne

Soit A, un gel à base de NaHA réticulé (Masse Molaire NaHA = 2,5×10⁶ Da, concentration en NaHA = 22,5 mg/ml, Taux de réticulation = 9%). Le gel est ensuite purifié par dialyse pendant 24 heures (cellulose régénérée, limite de séparation : Masse Molaire = 60kDa). 150g de gel purifié est mélangé 10 minutes à la spatule.

Le gel ainsi obtenu est divisé en 3 fractions de masse égale (50g).

Soit B, la fraction n°1. Dans cette fraction, on ajoute 1 % de glycérol, 1,5% de sorbitol et 0,3% de lidocaïne. Le gel est mélangé à la spatule pendant 10 minutes.

Soit C, la fraction n°2. Dans cette fraction, on ajoute 1 % de glycérol et 1,5% de sorbitol. Le gel est mélangé à la spatule pendant 10 minutes.

Soit D, la fraction n°3. On ajoute une solution de NaCl de concentration adaptée afin d'avoir une concentration en acide hyaluronique et une osmolarité équivalentes à celles des gels B et C. Le gel est mélangé à la spatule pendant 10 minutes.

Soient B, C et D, les gels issus des fractions B, C et D respectivement.

Les gels ainsi obtenus ont un pH proche de 7,00, une osmolarité proche de 300 mOsm/kg et une concentration en acide hyaluronique équivalente.

Une mesure de rhéologie (balayage en fréquence - 0,01 à 100 Hz) est effectuée pour chacun des gels B, C et D avant stérilisation.

Une comparaison des valeurs de G' (= module élastique), G" (= module visqueux) et Tanδ=G"/G' est effectuée à 1 Hz.

| Formulation | G'(1Hz) (Pa) | G"(1Hz) (Pa) | Tanδ(1 Hz) |
|---|---|---|---|
| Gel B (avant stérilisation) Selon l'invention | 316 | 94 | 0,297 |
| Gel C (avant stérilisation) | 314 | 93 | 0,296 |
| Gel D (avant stérilisation) | 318 | 94 | 0,296 |

On ne constate pas de différences rhéologiques, donc de différence de structure entre les 3 gels B, C et D avant stérilisation.

Les gels B, C et D sont remplis en seringue verre 1ml puis stérilisés à la chaleur à 121°C pendant 10 minutes.

Une mesure de rhéologie (balayage en fréquence - 0,01 à 100 Hz) est effectuée pour chacun des gels B, C et D après stérilisation.

Une comparaison des valeurs de G' (= module élastique), G" (= module visqueux) et Tanδ=G"/G' est effectuée à 1 Hz.

| Formulation | G'(1Hz) (Pa) | G"(1Hz) (Pa) | Tanδ(1 Hz) |
|---|---|---|---|
| Gel B (après stérilisation) Selon l'invention | 128 | 62 | 0,484 |
| Gel C (après stérilisation) | 91 | 47 | 0,516 |
| Gel D (après stérilisation) | 75 | 50 | 0,667 |

On constate des différences rhéologiques importantes, donc des différences importantes de structure entre les 3 gels B, C et D après stérilisation à la chaleur.

Après stérilisation à la chaleur, le gel selon l'invention (= gel B) présente une élasticité (G' plus élevé) et un caractère élastique (Tanδ plus faible) significativement plus important que le gel sans polyol et sans lidocaïne (= gel D).

La présence des polyols et de la lidocaïne a permis de fortement limiter la dégradation thermique du gel à base d'acide hyaluronique au cours de la stérilisation à la chaleur.

| Formulation | Variation G'(1Hz) avant/après stérilisation |
|---|---|
| Gel B Selon l'invention | - 59% |
| Gel C | -71% |
| Gel D | - 76% |

### Exemple 2 : Mise en évidence de la meilleure résistance à la dégradation enzymatique d'un gel à base d'acide hyaluronique avec polyol et lidocaïne.

La résistance à la dégradation enzymatique du gel B selon l'invention (voir exemple 1) est comparée à celle du gel D (voir exemple 1).

Le test de dégradation est effectué à l'aide d'un rhéomètre AR2000 (TA instruments) avec une géométrie plate de 40 mm et un entrefer de 1000 microns.

Le test de dégradation est effectué en ajoutant une solution de hyaluronidases dans le gel à tester, en homogénéisant à la spatule pendant 1 minute, en se plaçant à la température de 37°C et en imposant une déformation de 0,3%. La valeur du paramètre G' à 1Hz est mesurée à t=5 min et t=40 minutes.

| Formulation | G'(1Hz) (Pa) A t = 5 min | G'(1Hz) (Pa) A t = 40 min | ΔG'(1 Hz) |
|---|---|---|---|
| Gel B (stérile) Selon l'invention | 115 | 52 | - 55% |
| Gel D (stérile) | 60 | 24 | - 60% |

On constate que le gel selon l'invention a une meilleure résistance à la dégradation enzymatique.

### Exemple 3 : Mise en évidence de la meilleure résistance à la dégradation radicalaire d'un gel à base d'acide hyaluronique avec polyol et lidocaïne.

La résistance à la dégradation radicalaire du gel B selon l'invention (voir exemple 1), du gel C (voir exemple 1) et du gel D (voir exemple 1) est comparée.

Le test de dégradation est effectué à l'aide d'un rhéomètre AR2000 (TA instruments) avec une géométrie plate de 40 mm et un entrefer de 1000 µm.

Le test de dégradation est effectué en ajoutant un oxydant dans le gel à tester, en homogénéisant à la spatule pendant 1 minute, en se plaçant à la température de 37°C et en imposant une déformation de 0,3%. La valeur du paramètre G' à 1 Hz est mesurée à t=5 min et t=40 min.

| Formulation | G'(1Hz) (Pa) A t = 5 min | G'(1Hz) (Pa) A t = 40 min | ΔG'(1 Hz) |
|---|---|---|---|
| Gel B (stérile) Selon l'invention | 122 | 98 | - 20% |
| Gel C (stérile) | 80 | 38 | - 53% |
| Gel D (stérile) | 58 | 20 | - 66% |

On constate que le gel selon l'invention possède une bien meilleure résistance à la dégradation radicalaire.

### Exemple 4 : Mise en évidence de la meilleure résistance à la dégradation thermique d'un gel à base d'acide hyaluronique avec polyol et lidocaïne

La résistance à la dégradation thermique du gel B selon l'invention (voir exemple 1) et du gel D (voir exemple 1) est comparée.

Le test de dégradation thermique est effectué en introduisant le gel à tester dans une étuve à 80°C pendant 8 jours et en mesurant la valeur du paramètre G'(1 Hz) à t=0 et à t=8 jours.

| Formulation | G'(1Hz) (Pa) A t = 0 | G'(1Hz) (Pa) A t = 8 jours | ΔG'(1 Hz) |
|---|---|---|---|
| Gel B (stérile) Selon l'invention | 128 | 66 | - 48% |
| Gel D (stérile) | 75 | 29 | -61% |

On constate que le gel selon l'invention a une meilleure résistance à la dégradation thermique.

### Exemple 5: Mise en évidence de la meilleure stabilité rhéologique à température ambiante au cours du temps d'un gel à base d'acide hyaluronique avec polyol et lidocaïne

Le gel B selon l'invention (voir exemple 1) et le gel D (voir exemple 1) ont été stockés 8 mois à température ambiante (25°C).

La valeur du paramètre G'(1 Hz) a été mesurée à t=0, t=4 mois et t=8 mois.

| Formulation | Gel B (stérile) Selon l'invention | Gel D (stérile) |
|---|---|---|
| G'(1Hz) (Pa) | 128 | 75 |
| A t = 0 | | |
| G'(1Hz) (Pa) | 126 | 68 |
| A t = 4 mois | | |
| G'(1Hz) (Pa) | 120 | 66 |
| A t = 8 mois | | |
| ΔG'(1 Hz) | -6% | -12% |
| (0 - 8 mois) | | |

On constate que le gel selon l'invention a une meilleure stabilité rhéologique à température ambiante au cours du temps.

## Revendications

1. Composition aqueuse injectable sous forme de gel, utilisée dans des buts esthétiques ou dans des buts thérapeutiques, à base d'acide hyaluronique réticulé ou l'un de ses sels, d'un ou plusieurs polyol(s) et de lidocaïne, ayant subi une stérilisation à la chaleur.

2. Composition aqueuse injectable selon la revendication 1, **caractérisée en ce que** la concentration en acide hyaluronique réticulé ou l'un de ses sels est comprise entre 0,01 mg/ml et 100 mg/ml, et la masse moléculaire de l'acide hyaluronique ou l'un de ses sels est comprise entre 1000 g/mol et 10×10⁶ g/mol.

3. Composition aqueuse injectable selon les revendications 1 ou 2, **caractérisée en ce que** la concentration en polyol est comprise entre 0,0001 et 100 mg/ml.

4. Composition aqueuse injectable l'une quelconque des revendications 1 à 3, **caractérisée en ce que** la concentration en lidocaïne est comprise entre 0,0001 et 50 mg/ml.

5. Composition aqueuse injectable selon l'une quelconque des revendications 1 à 4, **caractérisée en ce que** le gel à base d'acide hyaluronique ou l'un de ses sels est réticulé avec ou sans un ou plusieurs autres polysaccharides d'origine naturelle.

6. Composition aqueuse injectable selon l'une quelconque des revendications 1 à 5, **caractérisée en ce que** le gel à base d'acide hyaluronique ou l'un de ses sels est réticulé par des molécules bi- ou poly-fonctionnelles choisies parmi les époxydes, les épihalohydrines et la divinylsulfone.

7. Composition aqueuse injectable selon la revendication 6, **caractérisée en ce que** le gel à base d'acide hyaluronique ou l'un de ses sels est réticulé par le butanediol diglycidyle éther (BDDE).

8. Composition aqueuse injectable selon l'une quelconque des revendications 1 à 7, **caractérisée en ce que** le polyol est choisi parmi le glycérol, le sorbitol, le mannitol, le propylène glycol, l'erythritol, le xylitol, le maltitol et le lactitol.

9. Composition aqueuse injectable selon l'une quelconque des revendications 1 à 8, utilisée pour le comblement ou le remplacement de tissus biologiques comme par exemple le comblement des rides, le remodelage du visage et l'augmentation du volume des lèvres.

10. Composition aqueuse injectable selon l'une quelconque des revendications 1 à 8, utilisée dans le traitement de réhydratation de la peau par mésothérapie.

11. Composition aqueuse injectable selon l'une quelconque des revendications 1 à 8, utilisée pour séparer, remplacer ou combler un tissu biologique ou augmenter le volume dudit tissu.

12. Composition aqueuse injectable selon l'une quelconque des revendications 1 à 8, utilisée :
- en rhumatologie, en tant qu'agent de remplacement, de supplément temporaire du liquide synovial ;
- en urologie/gynécologie, en tant qu'agent permettant d'augmenter le volume du sphincter ou de l'urètre ;
- en ophtalmologie, en tant qu'adjuvant à la chirurgie de la cataracte ou pour le traitement des glaucomes ;
- en pharmaceutique, en tant que gel à libération de substances actives ;
- en chirurgie, pour la reconstruction osseuse, l'augmentation du volume des cordes vocales ou la fabrication de tissus chirurgicaux.

13. Procédé d'amélioration des propriétés rhéologiques de viscoélasticité d'un gel à base d'acide hyaluronique réticulé ou l'un de ses sels, **caractérisé en ce qu'**il comprend l'ajout d'un ou plusieurs polyol(s) et de lidocaïne au dit gel, suivi d'une stérilisation à la chaleur, ayant pour effet une amélioration des propriétés rhéologiques de viscoélasticité du gel, par rapport à un gel sans polyol et sans lidocaïne.

14. Procédé d'amélioration de la stabilité rhéologique au cours du temps d'un gel à base d'acide hyaluronique réticulé ou l'un de ses sels, **caractérisé en ce qu'**il comprend l'ajout d'un ou plusieurs polyol(s) et de lidocaïne au dit gel, suivi d'une stérilisation à la chaleur, ayant pour effet une amélioration de la stabilité rhéologique au cours du temps du gel, par rapport à un gel sans polyol et sans lidocaïne.

15. Procédé d'amélioration de la rémanence in vivo d'un gel à base d'acide hyaluronique réticulé ou l'un de ses sels, **caractérisé en ce qu'**il comprend l'ajout d'un ou plusieurs polyol(s) et de lidocaïne au dit gel, suivi d'une stérilisation à la chaleur, ayant pour effet une amélioration de la rémanence in vivo du gel, par rapport à un gel sans polyol et sans lidocaïne.

## Patentansprüche

1. Injizierbare wässrige Zusammensetzung in Gelform, die zu ästhetischen Zwecken oder therapeutischen Zwecken verwendet wird, auf der Basis von vernetzter Hyaluronsäure oder eines ihrer Salze, eines oder mehrerer Polyole und von Lidocain, die mit Wärme sterilisiert wurde.

2. Injizierbare wässrige Zusammensetzung nach Anspruch 1, **dadurch gekennzeichnet, dass** die Konzentration der vernetzten Hyaluronsäure oder eines ihrer Salze zwischen 0,01 mg/ml und 100 mg/ml ist und die molekulare Masse der Hyaluronsäure oder eines ihrer Salze zwischen 1000 g/mol und 10 x 10⁶ g/mol ist.

3. Injizierbare wässrige Zusammensetzung nach Anspruch 1 oder 2, **dadurch gekennzeichnet, dass** die Polyolkonzentration zwischen 0,0001 und 100 mg/ml ist.

4. Injizierbare wässrige Zusammensetzung nach einem der Ansprüche 1 bis 3,
**dadurch gekennzeichnet, dass** die Lidocainkonzentration zwischen 0,0001 und 50 mg/ml ist.

5. Injizierbare wässrige Zusammensetzung nach einem der Ansprüche 1 bis 4,
**dadurch gekennzeichnet, dass** das Gel auf der Basis von Hyaluronsäure oder eines ihrer Salze mit oder ohne einem oder mehreren anderen natürlichen Polysacchariden vernetzt ist.

6. Injizierbare wässrige Zusammensetzung nach einem der Ansprüche 1 bis 5,
**dadurch gekennzeichnet, dass** das Gel auf der Basis von Hyaluronsäure oder eines ihrer Salze durch bi- oder multifunktionelle Moleküle vernetzt ist, die aus den Epoxyden, den Epihalohydrinen und dem Divinylsulfon ausgewählt sind.

7. Injizierbare wässrige Zusammensetzung nach Anspruch 6, **dadurch gekennzeichnet, dass** das Gel auf der Basis von Hyaluronsäure oder eines ihrer Salze durch Butandioldiglycidylether (BDDE) vernetzt ist.

8. Injizierbare wässrige Zusammensetzung nach einem der Ansprüche 1 bis 7,
**dadurch gekennzeichnet, dass** das Polyol aus Glycerol, dem Sorbitol, dem Mannitol, dem Propylenglycol, dem Erythritol, dem Xylitol, dem Maltitol und dem Lactitol ausgewählt ist.

9. Injizierbare wässrige Zusammensetzung nach einem der Ansprüche 1 bis 8, die zum Aufpolstern oder zum Ersatz biologischer Gewebe, wie beispielsweise zum Aufpolstern von Falten, zur Modellierung des Gesichts und zur Vergrößerung des Volumens der Lippen, verwendet wird.

10. Injizierbare wässrige Zusammensetzung nach einem der Ansprüche 1 bis 8, die für die Rehydrationsbehandlung der Haut durch Mesotherapie verwendet wird.

11. Injizierbare wässrige Zusammensetzung nach einem der Ansprüche 1 bis 8, die verwendet wird, um ein biologisches Gewebe zu separieren, zu ersetzen oder aufzupolstern oder um das Volumen des Gewebes zu vergrößern.

12. Injizierbare wässrige Zusammensetzung nach einem der Ansprüche 1 bis 8, die verwendet wird:
- in der Rheumatologie als Ersatz-, temporäres Ergänzungsmittel der Gelenkflüssigkeit,
- in der Urologie/Gynäkologie als Mittel, das es erlaubt, das Volumen des Schließmuskels oder der Harnröhre zu vergrößern,
- in der Augenheilkunde als Adjuvans in der Kataraktchirurgie oder zur Behandlung von Glaukomen,
- in der Pharmazie als Gel für die Freisetzung aktiver Substanzen,
- in der Chirurgie für die Wiederherstellung von Knochen, die Vergrößerung des Volumens der Stimmbänder oder die Herstellung von chirurgischen Geweben.

13. Verfahren zum Verbessern der rheologischen Viskoelastizitätseigenschaften eines Gels auf der Basis von vernetzter Hyaluronsäure oder eines ihrer Salze, **dadurch gekennzeichnet, dass** dieses die Zugabe eines oder mehrerer Polyole und Lidocain zu dem Gel umfasst, gefolgt von einer Sterilisation mit Wärme, was im Vergleich zu einem Gel ohne Polyol und ohne Lidocain eine Verbesserung der rheologischen Viskoelastizitätseigenschaften des Gels zur Folge hat.

14. Verfahren zum Verbessern der rheologischen Stabilität über die Zeit eines Gels auf der Basis von vernetzter Hyaluronsäure oder eines ihrer Salze, **dadurch gekennzeichnet, dass** dieses die Zugabe eines oder mehrerer Polyole und Lidocain zu dem Gel umfasst, gefolgt von einer Sterilisation mit Wärme, was im Vergleich zu einem Gel ohne Polyol und ohne Lidocain eine Verbesserung der rheologischen Stabilität des Gels zur Folge hat.

15. Verfahren zum Verbessern der *in vivo* Remanenz eines Gels auf der Basis von vernetzter Hyaluronsäure oder eines ihrer Salze, **dadurch gekennzeichnet, dass** dieses die Zugabe eines oder mehrerer Polyole und Lidocain zu dem Gel umfasst, gefolgt von einer Sterilisation mit Wärme, was im Vergleich zu einem Gel ohne Polyol und ohne Lidocain eine Verbesserung der *in vivo* Remanenz des Gels zur Folge hat.

## Claims

1. An injectable aqueous composition in .the form of a get, used for aesthetical pur poses or for therapeutic purposes, based on crosslinked hyaluronic acid or one of its salts, on one or more polyol(s) and on lidocaine, having undergone heat sterilization.

2. The injectable aqueous composition according to claim 1, **characterized in that** the concentration of crosslinked hyaluronic acid or of one of its salts is comprised between 0.01 mg/ml and 100 mg/ml, and the molecular mass of the hyaluronic acid or one of its salts is between 1000 g/mol and 10x10⁶ g/mol.

3. The injectable aqueous composition according to claim 1 or 2, **characterized in that** the polyol concentration is comprised between 0.0001 and 100 mg/ml.

4. The injectable aqueous composition according to any one of claims 1 to 3, **characterized in that** the lidocaine concentration is comprised between 0.0001 and 50 mg/ml.

5. The injectable aqueous composition according to any one of claims 1 to 4, **characterized in that** the gel based on hyaluronic acid or one of its salts is crosslinked with or without one or more other polysaccharides of natural origin.

6. The injectable aqueous composition according to any one of claims 1 to 5, **characterized in that** the gel based on hyaluronic acid or one of its salts is crosslinked with bi- or polyfunctional molecules selected from epoxides, epihalohydrins and divinylsulfone.

7. The injectable aqueous composition according to claim 6, **characterized in that** the gel based on hyaluronic acid or one of its salts is crosslinked with 1,4-butanediol diglycidyl ether (BDDE).

8. The injectable aqueous composition according to any one of claims 1 to 7, **characterized in that** the polyol is selected from glycerol, sorbitol, mannitol, propylene glycol, erythritol, xylitol, maltitol and lactitol.

9. The injectable aqueous composition according to any one of claim 1 to 8, used for filling or replacing biological tissues, such as the filling of wrinkles, the remodeling of the face and the increasing of the volume of the lips.

10. The injectable aqueous composition according to any one of claims 1 to 8, used in skin rehydration treatment by mesotherapy.

11. The injectable aqueous composition according to any one of claims 1 to 8, used for separating, replacing or filling a biological tissue or increasing the volume of said tissue.

12. The injectable aqueous composition according to any one of claims 1 to 8, used:
- in rheumatology, as a replacement agent, temporary supplemental agent of synovial fluid;
- in urology/gynecology, as an agent for increasing sphincter volume or urethral volume;
- in ophthalmology, as an adjuvant to cataract surgery or for treating glaucomas;
- in pharmaceutics, as a gel for releasing active substances;
- in surgery, for bone reconstruction, increasing the volume of the vocal cords or for manufacturing surgical tissues.

13. A process for improving the viscoelastic rheological properties of a gel based on crosslinked hyaluronic acid or one of its salts, **characterized in that** it comprises the addition of one or more polyol(s) and lidocaine to said gel, followed by heat sterilization, which has the effect of improving the viscoelastic rheological properties of the gel, as compared to a gel without polyol and without lidocaine.

14. A process for improving the rheological stability over time of a gel based on crosslinked hyaluronic acid or one of its salts, **characterized in that** it comprises the addition of one or more polyol(s) and lidocaine to said gel, followed by heat sterilization, which has the effect of improving the rheological stability over time of the gel, as compared to a gel without polyol and without lidocaine.

15. A process for improving the *in vivo* remanence of a gel based on crosslinked hyaluronic acid or one of its salts, **characterized in that** it comprises the addition of one or more polyol(s) and lidocaine to said gel, followed by heat sterilization, which has the effect of improving the *in vivo* remanence of the gel, as compared to a gel without polyol and without lidocaine.
